# EUROPEAN PATENT APPLICATION

(11) **EP 4 534 693 A1**
(43) Date of publication of application: **09.04.2025**
(21) Application number: 23811191.8
(22) Date of filing: 26.05.2023
(51) Int. Cl.: C12Q 1/6876, C12Q 1/6806, C12Q 1/6837, C12Q 1/686, C12Q 1/6851, G16B 25/20

(54) **APPLICATION OF CIRCULAR RNA IN PREPARATION OF CEREBRAL STROKE DIAGNOSIS PRODUCT**

(30) Priority: 26.05.2022 CN 202210582160
(71) Applicant: Orisomes Biotech Co., Ltd, Qingdao, Shandong 266000 (CN)
(72) Inventor: JI, Guangju, Qingdao, Shandong 266000 (CN); WANG, Huiwen, Qingdao, Shandong 266000 (CN); YANG, Zhiguang, Qingdao, Shandong 266000 (CN); BI, Youkun, Qingdao, Shandong 266000 (CN)
(74) Representative: Leonard, Thomas Charles
(86) International application number: PCT/CN2023/096620
(87) International publication number: WO 2023/227121

(57) **Abstract**

Provided is an application of a circular RNA in the preparation of a cerebral stroke diagnosis product, belonging to the field of circular RNA medical treatments. Provided is a kit, comprising a reagent and/or test paper and/or gene chip for detecting a circular RNA-circ-Magi1 . The nucleotide sequence of the circular RNA-circ-Magi1 is as shown in SEQ ID NO: 1 or SEQ ID NO: 4. Further provided is the application of a detection system for the diagnosis, assisted diagnosis or prognosis evaluation of cerebral stroke, and a detection reagent of the circular RNA-circ-Magi1 in the preparation of a kit, test paper, chip or high-throughput sequencing platform for the diagnosis, assisted diagnosis or prognosis evaluation of cerebral stroke.

## Description

### CROSS REFERENCE TO RELATED APPLICATIONS

This application claims the priority of the Chinese patent application No. 2022105821606, titled "APPLICATION OF CIRCULAR RNA IN PREPARATION OF CEREBRAL STROKE DIAGNOSIS PRODUCT", filed on May 26, 2022, which is incorporated herein by reference in its entirety.

### TECHNICAL FIELD

The present disclosure relates to the medical field of circular RNA, in particular to a circular RNA-circ-Magi1, a kit for diagnosis, auxiliary diagnosis or prognosis evaluation of cerebral stroke, a detection system for diagnosis, auxiliary diagnosis or prognosis evaluation of cerebral stroke, and use of a detection reagent for circular RNA -circ- Magi 1 in preparing kits, test strips, chips or high-throughput sequencing platforms for diagnosis, auxiliary diagnosis or prognosis evaluation of cerebral stroke.

### BACKGROUND

Cerebral stroke, also known as stroke, is an acute cerebrovascular disease caused by brain tissue damage due to rupture or blockage of blood vessels in the brain which results in that blood cannot flow to the brain, including ischemic and hemorrhagic strokes. Cerebral stroke has the characteristics of high morbidity, high mortality, high disability rate, high recurrence rate, etc. Cerebral stroke has become the leading cause of death in China as well as the primary cause of disability among Chinese adults.

Currently, conventional diagnosis for cerebral stroke mainly relies on clinical symptoms supplemented by imaging techniques and blood tests. Blood molecular markers are increasingly becoming an important indicator to help diagnosis.

With the deepening of genomics research, more and more non-coding RNAs have been identified, and their important role in the regulation of gene expression is being recognized. Circular RNA (circRNA), a special class of non-coding RNA, is a non-coding RNA that is covalently closed and looped, which is formed during reverse splicing. In eukaryotes, circular RNAs are expressed abundantly, widely existing in various organs and tissues, exhibit spatiotemporal specificity and tissue specificity, and are also evolutionarily conserved. Circular RNAs, without 5' cap structure and 3' polyadenylate tail structure, are a closed loop in structure, which is not easily degraded by exonucleases. Therefore, the half-life of most circRNAs exceeds 48 hours, whereas the half-life of mRNA is only 10 hours on average.

It has been reported that circular RNAs are widely involved in the occurrence and development of various diseases, providing theoretical support for the elucidation of the mechanism of the occurrence and development of the diseases, and providing a new research idea for the diagnosis and treatment of various diseases. Circular RNAs have the characteristics of tissue specificity, disease specificity, timing specificity, and high stability, exhibiting an excellent application prospect in terms of diagnostic markers for diseases.

The clinical diagnosis of cerebral stroke is mainly performed by computed tomography (CT) and magnetic resonance imaging (MRI), but both CT and MRI have some limitations in the diagnosis of cerebral stroke. First of all, CT examinations are not sufficiently effective in judging early ischemic stroke. Within 24 hours after the onset of cerebral infarction but before the infarction is fully formed, CT scans often fail to find obvious abnormalities. Moreover, the large radiation dose of CT will cause certain harm to the human body. At present, there is no blood test indicator that can effectively diagnose cerebral stroke. For example, for myocardial infarction, blood test needs to be supplemented with cardiac troponin T (cTNT) and other indicators in addition to echocardiography. MRI can make up for the defects of CT to a certain extent in the early diagnosis of stroke. However, MRI is not suitable for patients with cardiac pacemakers and magnetic metal in their bodies due to its working principle, and MRI is also relatively expensive.

Therefore, finding a fast, accurate, and cheap method for diagnosing cerebral stroke has become an important field to be solved.

### SUMMARY

### OBJECTIVES OF THE INVENTION

An object of the present disclosure is to provide a circular RNA-circ-Magi1, a kit for diagnosis, auxiliary diagnosis or prognosis evaluation of cerebral stroke, a detection system for diagnosis, auxiliary diagnosis or prognosis evaluation of cerebral stroke, and use of a detection reagent for circular RNA-circ-Magi1 in preparing kits, test strips, chips or high-throughput sequencing platforms for diagnosis, auxiliary diagnosis or prognosis evaluation of cerebral stroke.

Patients suspected of having cerebral stroke can be directly drawn for blood tests after admission. The expression level of circular RNA-circ-Magi1 in blood or other tissues is analyzed so as to diagnose or assist in the diagnosis of cerebral stroke, with improved accuracy of early diagnosis of cerebral stroke.

### TECHNICAL SOLUTIONS

To achieve the above objective, according to various embodiments of the present disclosure, the present disclosure provides the following technical solutions.

In a first aspect, the present disclosure provides a kit for diagnosis, auxiliary diagnosis or prognosis evaluation of cerebral stroke, including a reagent and/or a test strip and/or a gene chip for detecting a circular RNA-circ-Magi1, optionally, wherein the circular RNA-circ-Magi1 has a nucleotide sequence as set forth in SEQ ID NO: 1. Specifically, SEQ ID NO: 1 is as follows.

GCACAACCCGATCTCCCAGAGAAGGAGAAGTGCCTGGCGTGGACTATAACTT
TCTGACTGTGAAGGAGTTCTTGGACCTCGAGCAGAGTGGGACTCTTCTGGAAGTCGGC
ACCTATGAAGGAAACTATTATGGGACACCCAAGCCTCCTAGCCAGCCAGTCAGTGGGAA
AGTGATCACGACGGATGCCTTGCACAGCCTTCAGTCTGGCTCTAAGCAGTCGACCCCGA
AGCGAACCAAGTCCTACAATGATATGCAAAATGCTGGCATAGTCCACGCGGAGAATGAG
GAGGAGGATGACGTTCCTGAAATGAACAGCAGCTTTACAG

Further, the detection reagent includes detection primers and/or probes, wherein the detection primers are primers for amplifying the circular RNA-circ-Magi1; optionally, the detection primers include sequences as set forth in SEQ ID NO: 2 and SEQ ID NO: 3.

Further, the detection primers are primers for amplifying the circular RNA-circ-Magi1, including sequences as set forth in SEQ ID NO: 2 and SEQ ID NO: 3, specifically:
TCTGCTCGAGGTCCAAGAAC (SEQ ID NO: 2);
CAGCAGCTTTACAGGCACAACC (SEQ ID NO: 3).

Further, the detection primers are a set of fluorescent quantitative PCR primers for detecting the expression level of circular RNA-circ-Magi1; optionally, the reagent further includes a reagent for real-time quantitative PCR.

Further, a RNA extraction reagent is also included.

Further, a cDNA reverse transcription reagent is also included.

Further, the cerebral stroke is one or more of ischemic cerebral stroke, hemorrhagic cerebral stroke and lacunar cerebral stroke.

In a second aspect, the present disclosure provides a detection system for diagnosis, auxiliary diagnosis or prognosis evaluation of cerebral stroke, including a detection system for quantitative or relative quantitative detection of the expression level of circular RNA-circ-Magi1, and an analysis system. The analysis system is configured to diagnose or predict cerebral stroke according to the detected expression level of the circular RNA-circ-Magi1, and is stored with a scoring model for predicting or evaluating cerebral stroke.

A possible scoring model is as follows: the expression level of the circular RNA-circ-Magi1 higher than a set value P is indicative of positive for cerebral stroke; and the expression level of the circular RNA-circ-Magi1 lower than the set value P is indicative of negative for cerebral stroke.

Optionally, the circular RNA-circ-Magi1 is expressed at a high level in blood of a patient with cerebral stroke.

Further, the circular RNA-circ-Magi1 has a nucleotide sequence as set forth in SEQ ID NO: 1.

Further, the detection system includes a kit according to any one of claims 3 to 5.

In a third aspect, the present disclosure provides use of a detection reagent for circular RNA-circ-Magi1 in preparing kits, test strips, chips or high-throughput sequencing platforms for diagnosis, auxiliary diagnosis or prognosis evaluation of cerebral stroke. Correspondingly, it can correspond to a method for diagnosis, auxiliary diagnosis or prognosis evaluation of cerebral stroke, including detecting a content of circular RNA-circ-Magi1 in blood through kits, test strips, chips or high-throughput sequencing platforms to diagnose or predict cerebral stroke.

Further, the circular RNA-circ-Magi1 has a nucleotide sequence as set forth in SEQ ID NO: 1.

Further, the detection reagent includes detection primers for detecting the expression level of the circular RNA-circ-Magi1, wherein the detection primers include sequences as set forth in SEQ ID NO: 2 and SEQ ID NO: 3.

Further, the cerebral stroke is one or more of ischemic cerebral stroke, hemorrhagic cerebral stroke and lacunar cerebral stroke.

Further, a sample to be detected for circular RNA-circ-Magi1 is blood.

Further, the expression level is determined by detecting the amount of the RNA-circ-Magi1 present in the blood; optionally, the circular RNA-circ-Magi1 is expressed at a high level in the blood of patients with cerebral stroke.

In a fourth aspect, the present disclosure provides a method for diagnosis, auxiliary diagnosis or prognosis evaluation of cerebral stroke, including detecting the expression level of circular RNA-circ-Magi1 in a sample to be tested using the kit as described above; or
detecting the expression level of circular RNA-circ-Magi1 quantitatively or relatively quantitatively using the detection system as described above, and diagnosing or predicting cerebral stroke according to the detected expression level of circular RNA-circ-Magi1.

Further, the sample to be tested is whole blood, serum, or plasma.

Further, the cerebral stroke is one or more of ischemic cerebral stroke, hemorrhagic cerebral stroke and lacunar cerebral stroke.

Further, the expression level is determined by detecting the amount of the RNA-circ-Magi1 present in blood; optionally, the circular RNA-circ-Magi1 is expressed at a high level in blood of patients with cerebral stroke.

One or more embodiments of the present disclosure will be described below in detail. Other features, objects and advantages of the present disclosure will be apparent from the description and claims.

### BRIEF DESCRIPTION OF THE ACCOMPANYING DRAWINGS

In order to better describe and illustrate embodiments and/or illustrations of those inventions disclosed herein, reference may be made to one or more of the accompanying drawings. The additional details or examples used to describe the accompanying drawings should not be considered as limitations to the scope of any of the disclosed inventions, the presently described embodiments and/or examples, and the presently understood best mode of these inventions.
FIG. 1 shows the relative expression levels of circular RNA circ-Magi1 in various organs of mice in Example 1 of the present disclosure.
FIG. 2 shows the relative expression levels of circular RNA circ-Magi1 in various parts of mouse brain tissue in Example 1 of the present disclosure.
FIG. 3 shows the expression levels of circular RNA circ-Magi1 in HT22 cell line during oxygen-glucose deprivation and during reoxygenation and glucose resupply in Example 2 of the present disclosure.
FIG. 4 shows the relative expression levels of circular RNA circ-Magi1 in H9c2 cell line during oxygen-glucose deprivation and during reoxygenation and glucose resupply in Example 2 of the present disclosure.
FIG. 5 shows the neurobehavioral score of MACO model mice in Example 3 of the present disclosure.
FIG. 6 shows the TTC staining result of the brain of MACO model mice in Example 3 of the present disclosure.
FIG. 7 shows the statistical analysis of the TTC staining result of the brain of MACO model mice in Example 3 of the present disclosure.
FIG. 8 shows the expression level of circular RNA circ-Magi1 in brain of MCAO mice at 24 hours post operation in Example 4 of the present disclosure.
FIG. 9 shows the change in expression level of circular RNA circ-Magi1 in blood of MCAO mice at 24 hours post operation in Example 4 of the present disclosure.
FIG. 10 shows the relationship between the expression level of circular RNA circ-Magi1 in blood and the cerebral ischemia area of MCAO mice at 24 hours post operation in Example 4 of the present disclosure.
FIG. 11 shows the expression levels of circ-Magi1 in blood and blood cells of the control group and the MCAO model group in Example 4 of the present disclosure.
FIG. 12 shows the ROC curve of the mouse cerebral stroke in Example 4 of the present disclosure.
FIG. 13 shows the expression level of circular RNA circ-Magi1 in the blood of patients and healthy people in Example 5 of the present disclosure.
FIG. 14 shows the relationship between the infarct area of the human cerebral stroke and the relative expression level of circular RNA circ-Magi1 in Example 5 of the present disclosure.
FIG. 15 shows the ROC curve of the mouse cerebral stroke in Example 5 of the present disclosure.

### DETAILED DESCRIPTION

In order to make the purpose, technical solutions and advantages of the embodiments of the present disclosure clearer, the technical solutions in the embodiments of the present application will be clearly and completely described below. Apparently, the described embodiments are part, but not all, of the embodiments of the present disclosure. Based on the embodiments in this application, all other embodiments obtained by persons of ordinary skill in the art without creative efforts fall within the protection scope of the present disclosure. Unless otherwise clearly defined, in the whole description and claims, the terms "comprise" or its variations such as "contain" or "include", etc. will be understood as including the elements or component parts as stated without excluding other elements or other component parts.

In addition, numerous specific details are given in the following detailed description in order to better illustrate the present application. It will be understood by those skilled in the art that the present application may be practiced without certain of the specific details. In some embodiments, materials, components, methods, means, and so on that are well known to those skilled in the art are not described in detail, so as to highlight the gist of the present disclosure.

Hereinafter, the present disclosure will be described in detail.

Magi1 protein belongs to the membrane-associated guanosine kinase family. It participates in the assembly process of polymeric protein complexes and locates at cell junctions, playing a role as a skeleton protein in the establishment of cell junctions, cell shape maintenance and cell signal transduction. Magi1 gene can form various mRNAs through alternative splicing during the transcription process, which in turn encodes various subtypes of Magi1 . Circ-Magi1 is a circular RNA molecule formed by reverse splicing of exon3 and exon4 of the Magi1 gene. The molecular characteristics and biological functions of circ-Magi1 have not been reported yet.

In the present disclosure, through the qPCR method, it was confirmed that circular RNA circ-Magi1, hereinafter referred to as circ-Magi1, was present in the mouse hippocampal neuron cell line HT22. Oxygen-glucose deprivation induced the expression of circ-Magi1, and reoxygenation and glucose resupply induced the further expression of circ-Magi1, showing that the expression of circ-Magi1 in nerve cells is significantly increased under the condition of oxygen-glucose deprivation. However, the expression of circular RNA circ-Magi1 did not change significantly in the rat cardiomyocyte cell line H9c2 under hypoxic conditions, indicating that the expression of circ-Magi1 induced under oxygen-glucose deprivation is specific to nerve cells. The expression level of circ-Magi1 in plasma of MCAO model was increased but not in blood cells, which may indicate that the change of expression of circular RNA circ-Magi1 in blood is due to the release of circ-Magi1 into the blood caused by nerve cell injury. In the blood of established mouse MCAO animal model and the blood sample of patients with cerebral stroke, the expression level of circ-Magi1 was positively correlated with the cerebral ischemia area, indicating that the expression level of circ-Magi1 is positively correlated with MCAO cerebral stroke, and thus circular RNA circ-Magi1 can be used as a biomarker for cerebral stroke.

Inventors of the present disclosure also collected blood samples from ischemic cerebral stroke patients after diagnosed with the cerebral stroke. Compared with the blood samples of healthy people, the expression level of circ-Magi1 in the blood of patients with cerebral stroke was significantly increased, indicating that circular RNA circ-Magi1 can be used as a biomarker for cerebral stroke. Therefore, a fast, accurate and cheap method is provided for the diagnosis or auxiliary diagnosis of cerebral stroke, which can improve the accuracy of early diagnosis of cerebral stroke, so as not to miss the prime time for treatment.

The present disclosure also provides a new idea for the prevention and treatment of cerebral stroke.

The present disclosure involves the following nucleotide sequences:
Human circ-Magi1 sequence (SEQ ID NO: 1): 
GCACAACCCGATCTCCCAGAGAAGGAGAAGTGCCTGGCGTGGACTATAACTT
TCTGACTGTGAAGGAGTTCTTGGACCTCGAGCAGAGTGGGACTCTTCTGGAAGTCGGC
ACCTATGAAGGAAACTATTATGGGACACCCAAGCCTCCTAGCCAGCCAGTCAGTGGGAA
AGTGATCACGACGGATGCCTTGCACAGCCTTCAGTCTGGCTCTAAGCAGTCGACCCCGA
AGCGAACCAAGTCCTACAATGATATGCAAAATGCTGGCATAGTCCACGCGGAGAATGAG
GAGGAGGATGACGTTCCTGAAATGAACAGCAGCTTTACAG
Human circ-Magi1 qPCR primers:

| | |
|---|---|
| h-circ-Magi1 (F) (SEQ ID NO: 2) | TCTGCTCGAGGTCCAAGAAC |
| h-circ-Magi1 (R) (SEQ ID NO: 3) | CAGCAGCTTTACAGGCACAACC |

Mouse circ-Magi1 sequence (SEQ ID NO: 4): 
GCACAACCCGGTCTCCCAGAGAAGGAGAAGTGCCTGGTGTGGATTACAGCTT
TCTGACTGTGAAGGAGTTCTTGGACCTCGAGCAGAGCGGGACCCTGTTGGAAGTCGGC
ACCTATGAAGGAAACTATTATGGGACACCCAAACCTCCTAGCCAGCCAGTCAGTGGGAA
AGTGATCACGACGGATGCCTTGCACAGCCTGCAGTCTGGCTCCAAGCAGTCGACCCCTA
AGCGAACAAAGTCCTACAATGATATGCAAAATGCTGGCATAGTCCACCCGGAGAATGAG
GAGGAGGAGGATGTCCCTGAAATGAACAGTAGCTTTACAG
Mouse circ-Magi1 qPCR primers:

| | |
|---|---|
| m-circ-Magi1 (F) (SEQ ID NO: 5) | TCTGCTCGAGGTCCAAGAAC |
| m-circ-Magi1 (R) (SEQ ID NO: 6) | CAGTAGCTTTACAGGCACAACC |

The human circ-Magi1 qPCR primers and the mouse circ-Magi1 qPCR primers were obtained through artificial synthesis.

The cell lines cultivated in the present disclosure are those commercially available. For example, mouse hippocampal neuron cell line HT22 and rat cardiomyocyte cell line H9c2 are commercially available products. Those cell lines from ATCC cell bank can be used.

All of the biological materials, such as mice, used in the present disclosure are commercially available. For example, C57BL male black mice were purchased from Beijing Vital River Laboratory Animal Technology Co., Ltd.

### Example 1 High expression of circ-Magi1 in mouse brain tissue

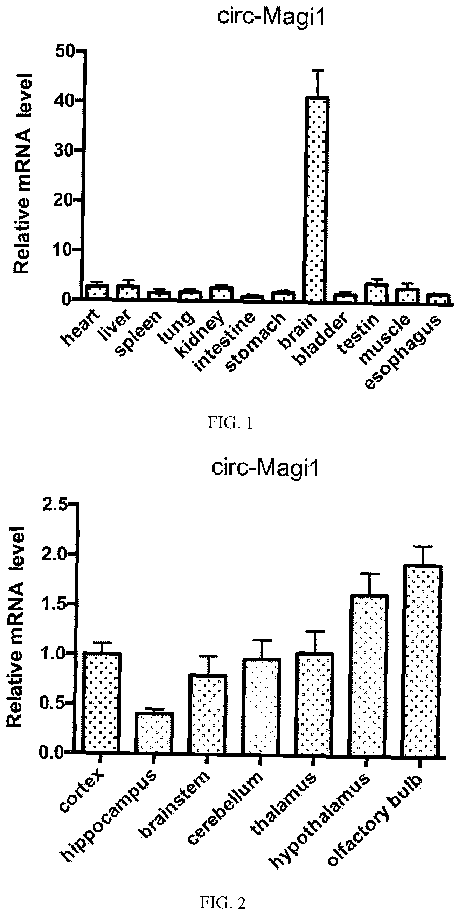

Five 8-week-old C57BL male black mice were selected and sacrificed by cervical dislocation. The heart, liver, spleen, lung, kidney, small intestine, stomach, brain, bladder, testis, skeletal muscle, esophagus and other tissues and organs were collected. RNA was extracted, and the expression of circ-Magi1 was measured with mouse circ-Magi1 qPCR primers by using the qPCR method. The result was shown in FIG.1, showing that circ-Magi1 was highly expressed in the brain tissue (i.e., a mixture of various parts of the brain).

For further analyzing the expression of circ-Magi1 in various parts of the brain, another five 8-week-old C57BL male black mice were sacrificed by cervical dislocation and then the following brain structures were collected: cerebral cortex, hippocampus, brainstem, cerebellum, thalamus, hypothalamus, and olfactory bulb. RNA was extracted, and the expression of circ-Magi1 was detected with mouse circ-Magi1 qPCR primers by using the qPCR method. The result was shown in FIG.2, showing a higher expression level in the hypothalamus and olfactory bulb, and a lower expression level in the hippocampus.

### Example 2 Expression of circ-Magi1 in mouse nerve cells during oxygen-glucose deprivation and recovery

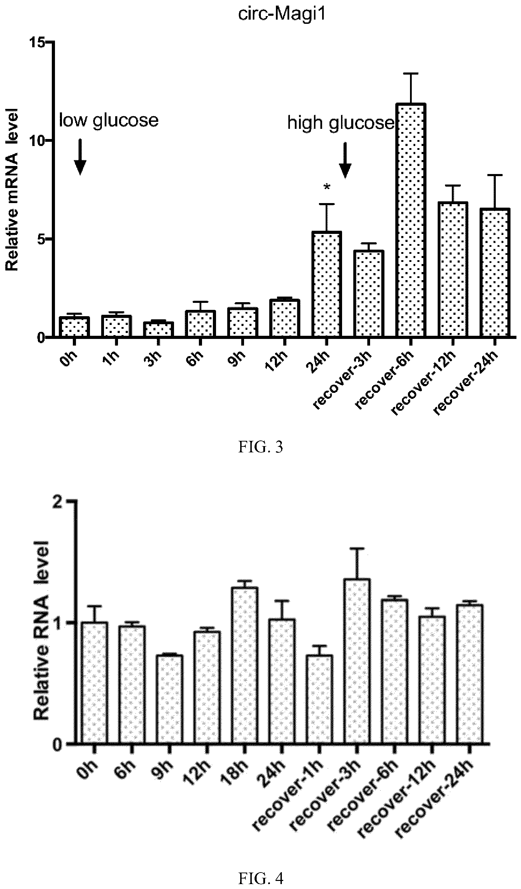

After the cells of mouse hippocampal neuron cell line HT22 were attached to the wall, the culture medium was replaced with a low-glucose medium, and the cells were cultured in a cell incubator with 1% O₂ and 5% CO₂ in saturated humidity for 24 hours. Then, the culture medium was replaced with a high-glucose medium, and the cells were cultured in the cell incubator with 5% CO₂ in saturated humidity for another 24 hours. The expression level of circ-Magi1 was measured at 1 hour, 3 hours, 6 hours, 9 hours, 12 hours, 24 hours after oxygen-glucose deprivation and at 3 hours, 6 hours, 12 hours, 24 hours after reoxygenation. As shown in FIG. 3, the oxygen-glucose deprivation induced the expression of circ-Magi1, and the recovery of oxygen and glucose further induced the expression of circ-Magi1 (n=3). That is, the expression of circ-Magi1 RNA in nerve cells was significantly increased under the condition of oxygen-glucose deprivation. The expression of circ-Magi1 RNA began to double at 12 hours, and reached 5 times at 24 hours, that is, the expression level could reach 2 to 5 times at 12 hours to 24 hours. The expression of circ-Magi1 RNA was further induced to increase after reoxygenation and glucose resupply.

The expression of circ-Magi1 RNA in rat cardiomyocyte cell line H9c2 under the condition of oxygen-glucose deprivation was further studied to verify the expression of circ-Magi1 in the cardiomyocyte cell line. The result was shown in FIG. 4, showing that the expression level of circ -Magi1 in the cardiomyocyte cell line H9c2 was not changed significantly under the hypoxic condition, indicating that hypoxia would not significantly change the expression level of circ-Magi1 in cardiomyocytes.

The result demonstrated that the expression of circ-Magi1 induced by oxygen-glucose deprivation was specific to nerve cells, suggesting that the change of expression level of circ-Magi1 in blood originates from nerve cells.

### Example 3 Establishment of mouse middle cerebral artery occlusion (MCAO) animal model

8-week-old C57BL male black mice were used, with three mice as a control. Eight mice were subjected to the following MCAO surgery for mouse. A 2 cm incision was made in the middle of the neck of mouse, and the right common carotid artery (CCA) was separated and ligated. Then upward, the external carotid artery (ECA) and the internal carotid artery (ICA) were separated. The ECA was ligated at proximal and distal ends, and the ICA was clamped with a vascular clip. A small incision was made between two lines that ligate the ECA to allow a filament to be inserted. The ECA was snipped and transposed. The filament was further inserted into the ICA about 9 mm deep. After 1 hour, the filament was withdrawn. The neck incision of the mouse was sutured.

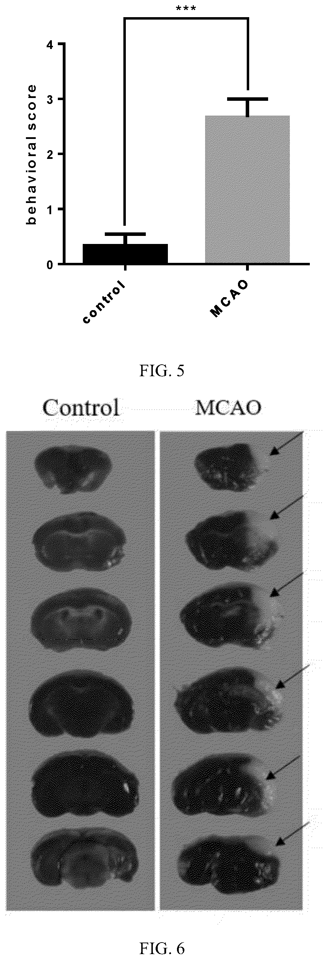

The above-mentioned MCAO mice were behaviorally scored at 24 hours post operation, and the result was shown in FIG. 5. The MACO animal model mice had obvious behavioral disorder, indicating that the MCAO animal model was successfully established.

The degree of cerebral infarction in MCAO model mice was determined by triphenyl tetrazolium chloride (TTC) staining.

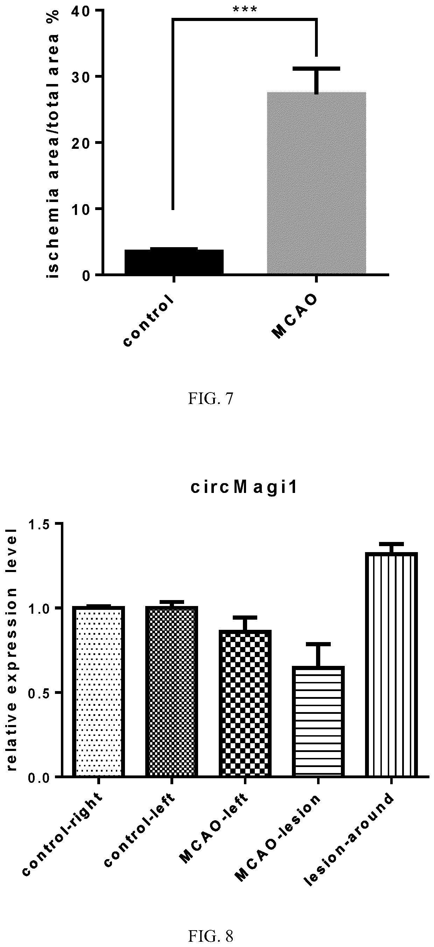

The process of TTC staining was as follows. The animals were sacrificed by brain decapitation. The brains were quickly removed within 10 minutes and placed in phosphate buffer saline (PBS) solution at 0°C to 4°C for transfer, and frozen in a refrigerator at -20°C for 30 minutes. The cerebellum, olfactory bulb, and lower brainstem were removed. The brains were sliced into coronal brain slices with a thickness of 2 mm, which were placed in 2% triphenyl tetrazolium chloride solution in water bath in dark at 37°C for 30 minutes. The containers were slightly shaken every 5 minutes to ensure fully staining. The brain slices were taken out and washed with PBS solution for 3 to 5 minutes. The brain slices can be photographed immediately or fixed with 10% neutral formaldehyde for 6 hours. The caudal side of each slice was selected for photographic analysis, and the result was shown in FIG. 6. The infarct area and total area of each slice were measured. The infarct volume of a slice is the product of the infarct area and the thickness of the slice. The sum of the infarct volumes of individual slices is the total infarct volume. The result was shown in FIG. 7.

The result showed that the infarct volume for the MCAO model accounted for about 28% of the total area, significantly higher than that for the control group. The model was successfully established.

### Example 4 Analysis of expression of circ-Magi1 in brain of MCAO animal model mice

The healthy mice and MCAO animal model mice were analyzed for the expression of circ-Magi1 in brain of the left and right brains of healthy mice, the left brain of MCAO mice, the lesion region of MCAO mice, and the lesion-around region of MCAO mice at 24 hours post operation by the qPCR method. The result was shown in FIG. 8, showing decreased expression of circ-Magi1 in the cerebral stroke lesion region, and increased expression in the lesion-around region.

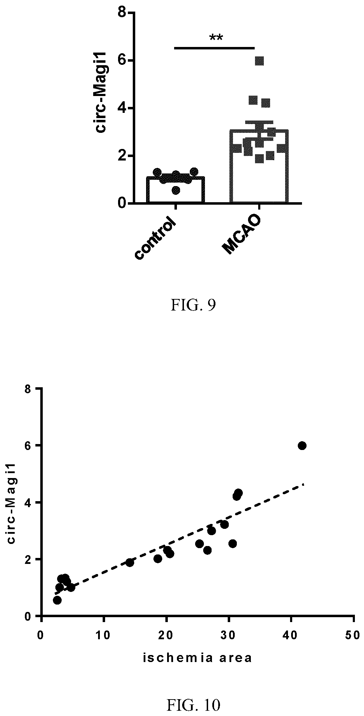

Circular RNA has a biological characteristic of high stability. The expression of RNA circ-Magi1 in the blood of healthy mice and MCAO animal model mice was detected at 24 hours post operation. The result was shown in FIG. 9, showing that the expression of circ-Magi1 in the blood of MCAO animal model mice was significantly increased. The relationship between the infarct area of mice and the expression level of RNA circ-Magi1 was analyzed. The result was shown in FIG. 10, showing that the expression level of RNA circ-Magi1 in blood was positively correlated with the degree of cerebral stroke in MCAO mice, with R² =0.8824 as shown in FIG. 10.

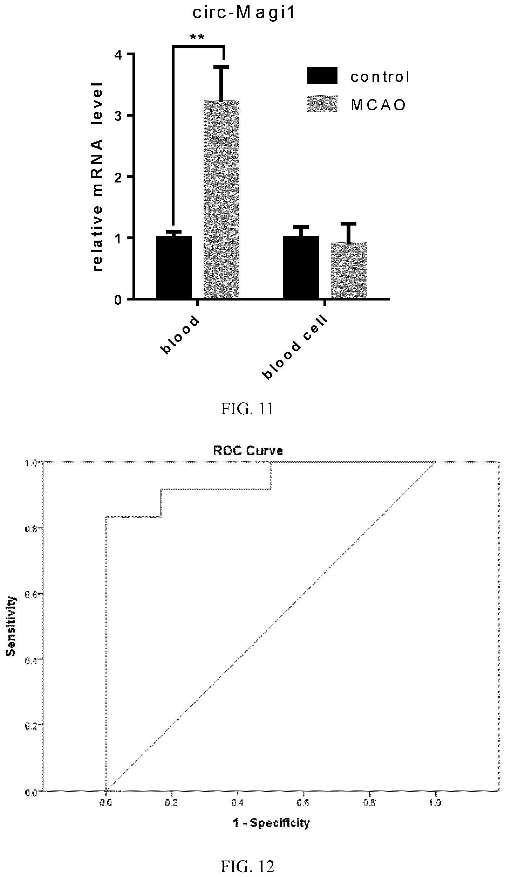

The expression of circ-Magi1 in blood plasma and blood cells of healthy mice and MCAO animal model mice was further analyzed. The result was shown in FIG. 11, showing that the expression level of circ-Magi1 in the blood cells of MCAO animal model mice was the same as that of healthy mice, and the expression level of RNA circ-Magi1 in the blood of MCAO animal model mice was significantly higher than that of healthy mice, indicating that the change in expression of circ-Magi1 in blood originates from cell-free circ-Magi1 in plasma.

Receiver operating characteristic curve (i.e., a ROC curve) is mainly used to evaluate the effect of an index on the classification/diagnosis of two types of subjects (such as patients and healthy people), and to find the best index threshold to achieve the best classification effect. For the ROC curve, the ordinate represents a true positive rate (TPR), and the abscissa represents a false positive rate (FPR). 1) True positive rate (TPR) is a ratio of the number of all actually positive samples that are correctly judged as positive to the number of all actually positive samples, and TPR is also called Sensitivity. 2) False positive rate (FPR) is a ratio of the number of all actually negative samples that are wrongly judged as positive to the number of all actually negative samples, and FPR is equal to 1-Specificity. The true positive rate reflects the degree of coverage of response that the index predicts, and the false positive rate reflects the degree of response that the index falsely reports. Therefore, a higher true positive rate and a lower false negative rate indicate a better degree of the index response. When it comes to a ROC graph, a steeper curve and more protrusion to the upper left indicate that the index is more responsive. The predictive effect of the index is evaluated by calculating the area under curve (AUC) value, and the ideal AUC value is 1.

12 MCAO model mice and 6 control mice were included. The ROC curve was plotted using SPSS. The result of the mouse ROC curve was shown in FIG. 12, showing that the area under curve (AUC) value was 0.944, and P=0.003 as shown in the table below. This suggests that the diagnostic accuracy was high, and that the degree of cerebral stroke can be diagnosed based on the expression level of RNA circ-Magi1 in blood.

| Area | Std. Error ^{a} | Asymptotic Sig. ^{b} | Asymptotic 95% Confidence Interval | |
|---|---|---|---|---|
| | | | Lower Bound | Upper Bound |
| .944 | .052 | .003 | .000 | 1.000 |

### Example 5 Analysis of expression of circ-Magi1 in patients with ischemic cerebral stroke

Blood samples were collected from patients with ischemic cerebral stroke within 24 hours after diagnosis of cerebral stroke. Collection of blood samples and CT examination were performed as soon as possible after the patients were admitted. For example, Wang Xquan, male, aged 83, admitted to the EICU Department of Puyang Oilfield General Hospital on March 20, 2021, was diagnosed with ischemic cerebral stroke by CT examination, belonging to the ischemic cerebral stroke patient group; Wang Xshan, male, aged 67, admitted to the emergency department of Puyang Oilfield General Hospital on April 17, 2021, was diagnosed with ischemic cerebral stroke by CT examination, belonging to the ischemic cerebral stroke patient group; and Li Xyu, male, aged 49, admitted to the emergency department of Puyang Oilfield General Hospital on April 17, 2021, was diagnosed with ischemic cerebral stroke by CT examination, belonging to the ischemic cerebral stroke patient group.

The control was blood samples from healthy people without cerebral stroke.

Samples were collected from Puyang Oilfield General Hospital with the consent of the Medical Ethics Committee of Puyang Oilfield General Hospital. All subjects who met the inclusion criteria in this study were informed and signed informed consent.

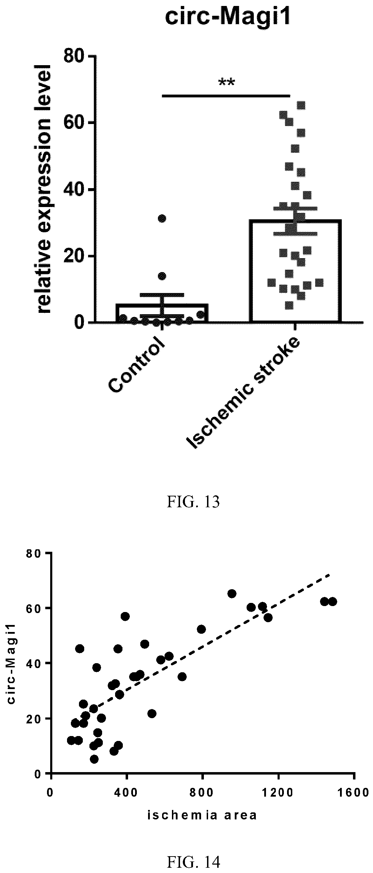

The expression of circ-Magi1 in the blood of patients with ischemic cerebral stroke was analyzed by the qPCR method. As shown in FIG. 13, it was found that the expression level of circ-Magi1 in the blood of patients with ischemic cerebral stroke was significantly higher than that of healthy people, which was about 25 times that in healthy people.

The relationship between the degree of cerebral stroke in MCAO people and the expression of circ-Magi1 in blood was analyzed. The result was shown in FIG. 14, showing that the expression of circ-Magi1 in blood was positively correlated with the degree of cerebral stroke in MCAO people. That is, the larger the infarct area, the higher the relative expression of circ-Magi1, with R² =0.6358.

By collecting blood samples immediately after admission, results can usually be obtained within one hour, which can improve the accuracy of early diagnosis and avoid missing the golden time for treatment.

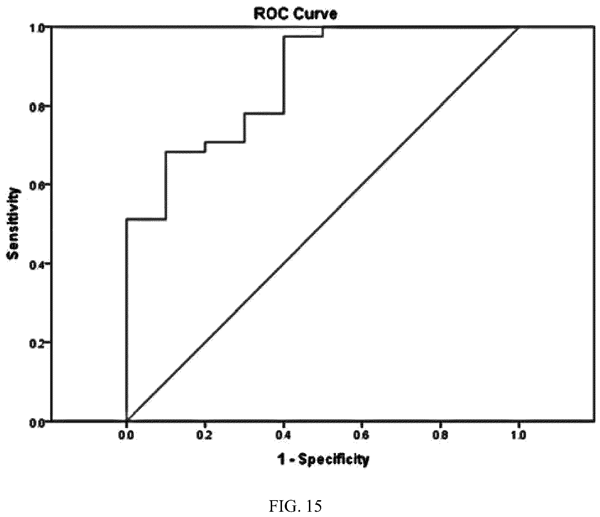

41 patients with ischemic cerebral stroke and 10 normal controls were included. A ROC curve was plotted using SPSS. The result of the ROC curve of patient's blood was shown in FIG. 15, showing that the area under curve (AUC) value was 0.866, and P=0.0003 as shown in the table below. This suggests that the diagnostic accuracy was high, and that the degree of cerebral stroke can be diagnosed based on the expression of RNA circ-Magi1 in blood.

| Area | Std. Error ^{a} | Asymptotic Sig. ^{b} | Asymptotic 95% Confidence Interval | |
|---|---|---|---|---|
| | | | Lower Bound | Upper Bound |
| .866 | .061 | .000 | .746 | .986 |

The above examples illustrate that the expression level of circular RNA circ-Magi1 in patients with cerebral stroke is significantly higher than that in healthy people, which can be used as an indicator for diagnosing stroke and also provides ideas for treating cerebral stroke by reducing the expression of circular RNA circ-Magi1.

Finally, it should be noted that the above embodiments are only used to illustrate the technical solutions of the present application, but not limit thereto. Although the present application has been described in detail with reference to the foregoing embodiments, those skilled in the art should understand that the technical solutions described in the foregoing embodiments can still be modified, or some technical features thereof can be equivalently replaced. However, these modifications or substitutions do not make the essence of the corresponding technical solutions deviate from the spirit and scope of the technical solutions of the embodiments of the present application.

## Claims

1. A kit for diagnosis, auxiliary diagnosis or prognosis evaluation of cerebral stroke, comprising a reagent and/or a test strip and/or a gene chip for detecting a circular RNA-circ-Magi1, optionally, wherein the circular RNA-circ-Magi1 has a nucleotide sequence as set forth in SEQ ID NO: 1.

2. The kit according to claim 1, wherein the detection reagent comprises detection primers and/or probes and/or a chip, wherein the detection primers are primers for amplifying the circular RNA-circ-Magi1;
optionally, the detection primers comprise sequences as set forth in SEQ ID NO: 2 and SEQ ID NO: 3;
optionally, the detection primers are a set of fluorescent quantitative PCR primers for detecting the expression level of the circular RNA-circ-Magi1;
optionally, the reagent further comprises a reagent for real-time quantitative PCR.

3. The kit according to claim 1 or 2, further comprising an RNA extraction reagent;
and/or a cDNA reverse transcription reagent.

4. The kit according to claim 2 or 3, wherein the cerebral stroke is one or more of ischemic cerebral stroke, hemorrhagic cerebral stroke and lacunar cerebral stroke.

5. A detection system for diagnosis, auxiliary diagnosis or prognosis evaluation of cerebral stroke, comprising a detection system for quantitative or relative quantitative detection of the expression level of circular RNA-circ-Magi1, and an analysis system;
the analysis system is configured to diagnose or predict cerebral stroke according to the detected expression level of the circular RNA-circ-Magi1, and is stored with a scoring model for predicting or evaluating cerebral stroke.

6. The detection system according to claim 5, wherein the circular RNA-circ-Magi1 has a nucleotide sequence as set forth in SEQ ID NO: 1.

7. The detection system according to claim 5 or 6, wherein the detection system comprises a kit according to any one of claims 3 to 5.

8. The detection system according to any one of claims 5 to 7, wherein the circular RNA-circ-Magi1 is expressed at a high level in blood of a patient with cerebral stroke;
and/or, the expression level of the circular RNA-circ-Magi1 higher than a set value P is indicative of positive for cerebral stroke; and the expression level of the circular RNA-circ-Magi1 lower than the set value P is indicative of negative for cerebral stroke.

9. Use of a detection reagent for circular RNA-circ-Magi1 in preparing kits, test strips, chips or high-throughput sequencing platforms for diagnosis, auxiliary diagnosis or prognosis evaluation of cerebral stroke.

10. The use according to claim 9, wherein the circular RNA-circ-Magi1 has a nucleotide sequence as set forth in SEQ ID NO: 1;
and/or the detection reagent comprises detection primers for detecting the expression level of the circular RNA-circ-Magi1, wherein the detection primers comprise sequences as set forth in SEQ ID NO: 2 and SEQ ID NO: 3;
and/or the cerebral stroke is one or more of ischemic cerebral stroke, hemorrhagic cerebral stroke and lacunar cerebral stroke;
and/or a sample to be detected for circular RNA-circ-Magi1 is whole blood, serum, or plasma;
and/or the expression level is determined by detecting the amount of the RNA-circ-Magi1 present in blood; optionally, the circular RNA-circ-Magi1 is expressed at a high level in blood of patients with cerebral stroke.

11. A method for diagnosis, auxiliary diagnosis or prognosis evaluation of cerebral stroke, comprising detecting the expression level of circular RNA-circ-Magi1 in a sample to be tested using the kit of any one of claims 1 to 4; or
detecting the expression level of circular RNA-circ-Magi1 quantitatively or relatively quantitatively using the detection system of any one of claims 5 to 8, and diagnosing or predicting cerebral stroke according to the detected expression level of circular RNA-circ-Magi1.

12. The method according to claim 11, wherein the sample to be tested is whole blood, serum, or plasma;
and/or the cerebral stroke is one or more of ischemic cerebral stroke, hemorrhagic cerebral stroke and lacunar cerebral stroke;
and/or the expression level is determined by detecting the amount of the RNA-circ-Magi1 present in blood; optionally, the circular RNA-circ-Magi1 is expressed at a high level in blood of patients with cerebral stroke.
